# EUROPÄISCHE PATENTANMELDUNG

(11) **EP 0 843 005 A2**
(43) Veröffentlichungstag der Anmeldung: **20.05.1998**
(21) Anmeldenummer: 97119900.5
(22) Anmeldetag: 13.11.1997
(51) Int. Cl.: C12N 5/08

(54) **Human-Coloncarcinom-Zellinien mit stabiler HSP72-Expression**

(30) Priorität: 15.11.1996 DE 19647426
(71) Anmelder: GSF-Forschungszentrum für Umwelt und Gesundheit, GmbH, 85764 Oberschleissheim (DE)
(72) Erfinder: Multhoff, Gabriele, 81675 München (DE)
(74) Vertreter: Reinhard - Skuhra - Weise & Partner

(57) **Zusammenfassung**

Erfindungsgemäß werden Human-Coloncarcinom-Zellinien bereitgestellt, die eine stabile HSP72-Expression von > 80% oder < 20% und ein im wesentlichen identisches Zelloberflächenproteinexpressionsmuster von MHC und Zelladhäsionsmolekülen zeigen.

## Beschreibung

Die Erfindung betrifft Human-Coloncarcinom-Zellinien mit einer stabilen HSP72-Expression.

Chaperone sind für eine Anzahl fundamentaler Prozesse notwendig, insbesondere, um dem Zellstreß entgegenzuwirken. Die am besten untersuchte Klasse von Chaperonen ist die Gruppe der Hitzeschock-Proteine (HSP) mit einem Molekulargewicht von 70 kDa. Sie binden intrazellulär an nicht native Konformationen anderer Proteine, stabilisieren sie und inhibieren auf diese Weise die Aggregation ungefalteter Proteine oder ermöglichen eine Transmembran-Translokation (1, 2). Es wurde beschrieben, daß HSP70 sowohl im Zellkern (3), im Cytosol und an der Zelloberfläche bestimmter Tumorzellen (4, 5) lokalisiert ist. Neben ihrer intrazellulären Aufgabe als Chaperone scheinen die Mitglieder der HSP70-Familie an der Stimulierung des Immunsystems beteiligt zu sein, d.h. bei der Entstehung bestimmter Autoimmunerkrankungen (6, 7), entzündlicher Prozesse unter Beteiligung von Pathogenen (8, 9) und bei der zellulären anti-Tumorimmunantwort in vivo und in vitro (10).

Die Gründe, weshalb hochkonservierte Proteine wie die Hitzeschockproteine immundominant sind, sind weiterhin unbekannt. Eine Zusammenfassung der zur Zeit diskutierten Hypothesen ist (11) zu finden.

Für alle immunstimulatorischen Funktionen scheint es notwendig zu sein, daß das HSP für immunkompetente Effektorzellen zugänglich ist. Untersuchungen zeigen, daß eine hitzeinduzierte Membran-Expression von HSP72 auf Human-Sarkomzellen mit einer erhöhten Lysesensitivität korrelliert, die durch natürliche Killerzellen (NK-Zellen) bewirkt wird. Die Tatsache, daS HSP72 nur auf der Zelloberfläche von Tumoren exprimiert wird, nicht dagegen auf normalen Zellen, zeigt, daß HSP als tumorspezifische Targetstrukturen für immunkompetente Effektorzellen wirken können.

Es ist eine Aufgabe der vorliegenden Erfindung, HSP72 exprimierende Zellinien bereitzustellen, die die Möglichkeit bieten, vergleichende Untersuchungen, beispielsweise über die Bedeutung von HSP72 für die Immunogenität von Tumorzellen, durchzuführen.

Diese Aufgabe wird erfindungsgemäß durch die im Anspruch 1 näher gekennzeichneten Merkmale gelöst. Bevorzugte Ausführungsformen der Erfindung ergeben sich aus den Unteransprüchen.

Zwei bevorzugte Zellinien wurden bei der DSM am 29. Oktober 1996 hinterlegt:

Eine Human-Coloncarcinom-Zellinie CX+ mit einer stabilen Expression von HSP72 an der Zelloberfläche von > 80%, bevorzugt > 90%, mit der Zugangsnummer DSM ACC 2287. Eine Human-Coloncarcinom-Zellinie CX⁻ mit einer stabilen Expression von HSP72 an der Zelloberfläche von < 20%, bevorzugt < 10%, mit der Zugangsnummer DSM ACC 2288.

Die Erfindung wird nachfolgend am Beispiel der Coloncarcinom-Zellinie CX2 näher erläutert. Sie ist jedoch nicht auf diese spezifische Zellinie beschränkt. Erfindungsgemäß können auch andere Coloncarcinom-Zellinien benutzt werden, um die gestellte Aufgabe zu lösen.

Erfindungsgemäß können beliebige Human-Coloncarcinom-Zellinien verwendet werden, die dem Fachmann bereits an sich bekannt sind oder die ohne weiteres erfinderisches Zutun durch an sich bekannte Techniken etablierbar sind. Die vorliegende Erfindung wurde am Beispiel zweier Human-Coloncarcinom-Zellinien durchgeführt, nämlich der CX2- und der HT29-Zellinien. Die CX2-Ausgangszellinie ist vom Deutschen Krebsforschungszentrum in Heidelberg erhältlich. Ein Tumorsteckbrief dieser Zellinie mit wichtigen genotypischen Parametern ist beigefügt. Gleiches gilt für die Zellinie HT29. Eine Hinterlegung gemäß dem Budapester Vertrag ist deshalb nicht erforderlich, da die Erfindung anhand der vorliegenden Literatur wiederholbar nacharbeitbar ist. Weiterhin können, wie bereits oben angeführt, beliebige andere Human-Coloncarcinom-Zellinien verwendet werden, um Sublinien zu erhalten, die eine stabile HSP72-Expression von > 80% oder < 20% und ein im wesentlichen identisches Zelloberflächen-Protein-Expressionsmuster von MHC und Zelladhäsionsmolekülen besitzen.

Nachfolgend wird die Erfindung unter Bezugnahme auf die beiliegenden Figuren näher erläutert. Die Figuren zeigen:

### Figur 1:

Kryo-Ultramikrotom-Abbildung von markiertem HSP72 auf CX2-Human-Coloncarcinomzellen. Die gefrorenen Schnitte wurden mit anti-HSP72 mAb (RPN1197, Amersham) und nachfolgend mit ultrakleinem Gold, das mit Silber verstärkt war, behandelt. Die Markierung von HSP72 ist nicht auf die subzellulären Teile im Zytoplasma und dem Kern beschränkt; HSP72 kann auch auf der Plasmamembran nachgewiesen werden, wie A und B zeigen. C zeigt eine Kontroll-Markierung mit Silberverstärkung ohne primären Antikörper. Der Pfeil zeigt die Anreicherung von HSP72 auf den Fortsätzen der Plasmamembran; N zeigt den Kern (x 43.000).

### Figur 2:

SDS-PAGE-Analyse von HSP72, das von der Zelloberfläche biotinylierter CX2-Tumorzellen immunpräzipitiert wurde.
Spur A: rHSP70-Protein (50 ng), nachgewiesen durch den spezifischen HSP72 mAb;
Spuren B und C: Immunpräzipitate, die mit anti-HSP72 (Spur B) oder anti-MHC Klasse I mAb (Spur C) auf 0,1% NP40 Lysaten nach selektiver Zelloberflächenbiotinylierung erhalten wurden. Linker Rand: Molekulargewichtsstandard.
Rechter Rand: Pfeile zeigen eine 72 KDa bzw. 45 kDa-Bande.

### Figur 3:

HSP72-Zelloberflächenexpression auf CX2-Coloncarcinomzellen nach durchflußzytometrischer Zellsortierung unter Verwendung eines HSP72 spezifischen mAb. Die Ergebnisse sind logarithmisch aufgetragen, und zwar die Intensität der grünen Fluoreszenz gegen die relative Zellzahl. Die gestrichelten Linien zeigen die Negativ-Kontrolle; die durchgezogene Linie HSP72 mAb.

### Figur 4:

HSP72-Immunblot von Zytoplasmalysaten von CX+, CX2 und CX- Carcinomzellen unter physiologischen Bedingungen (offene Balken) und nach einem milden Hitzestreß bei 41,8°C, 2 Stunden und nachfolgender Erholungsphase bei 37°C für 2 Stunden (schraffierte Balken). HSP72-Proteinmengen (mit einem Pfeil markiert) wurden auf Aktin (mit "a" bezeichnet) bezogen. Gleiche Proteinmengen (10 µg der Zellysate) wurden auf einer 10% SDS-PAGE unter reduzierenden Bedingungen elektrophoretisch getrennt und auf Nitrozellulose übertragen. Die 72 kDa-Proteinbande wurde mit dem HSP72 spezifischen mAb dargestellt und mit dem ECL-System nachgewiesen. Die Immunblots wurden mit Laser-Densitometrie quantifiziert. Die Ergebnisse zeigen den Mittelwert aus zumindest zwei unabhängigen Versuchen.
Einfügung: Ein repräsentativer Immunblot.
Die relative Rate der HSP72-Induktion ist unter jedem Balken angegeben.

### Figur 5:

Beispiel einer HSP72-Sortierung mit Einstellungsparametern.

### Figur 6 A-C:

### Beispiele einer HSP72-Sortierung von HT29-Zellen (FACS-Analysen)

Erfindungsgemäß konnte die Coloncarcinom-Mutter-Zellinie CX2 in eine Zellinie getrennt werden, die HSP72 in hoher Menge exprimiert (CX+: Expression > 80%, insbesondere > 90%) und eine HSP72 in geringer Menge exprimierende Zellinie (CX-: Expression < 20%, insbesondere < 10%). Diese Sublinien wurden durch Zellsortierung aufgrund der Expression von HSP72 an der Zelloberfläche mit Hilfe eines geeigneten Antikörpers gewonnen. Die bisher zur Verfügung stehenden menschlichen Tumorzellinien wiesen den Nachteil auf, daß sie Hitzeschockproteine über einen längeren Zeitraum nicht stabil exprimierten, d.h. nach einer bestimmten Zeit die HSP-Expression abnahm und erneut ein Hitzeschock notwendig war, um die Expression zu induzieren.

Erfindungsgemäß konnte gezeigt werden, daß Human-Coloncarcinom-Zellinien nach Zellsortierung der Ausgangszellinie mit Hilfe eines gegen HSP72 gerichteten monoklonalen Antikörpers in der Lage sind, HSP72 stabil zu exprimieren, wobei es überraschenderweise möglich war, durch Zellsortierung HSP72 in einer Menge von > 80% und in einer Menge von < 20% stabil exprimierende Zellinien zu erhalten.

Dieses Ergebnis war deshalb so unerwartet, da bei der Mutterzellinie ursprünglich eine über mehr als 20 getestete Zellpassagen stabile HSP72-Zelloberflächenexpression von > 60% nachweisbar war. Dieser Befund legt den Schluß nahe, daß diese 60 %-ige Expressionsrate beispielsweise für das Überleben, für den Zellzyklus, für die Zelladhäsion, usw. unverzichtbar ist.

Es war deshalb zu erwarten, daß nach Zellseparation von CX2-Zellen in eine HSP72 in hoher Menge exprimierende Sublinie und in eine HSP72 in geringer Menge exprimierende Sublinie sich beide Subzellinien nach wenigen Zellpassagen wieder bei einer ca. 60 %-igen HSP72-Zelloberflächenexpressionsrate einpendeln. Überraschenderweise war dies jedoch nicht der Fall. Beide Zellinien, CX+ und CX-, wiesen ein über mehr als 20 Zellpassagen stabiles Expressionsmuster der HSP72-Zelloberflächenexpression auf: CX+ > 80%, CX- < 20%.

Normalerweise weisen derartige Zellinien ein unterschiedliches Zelloberflächenproteinexpressionsmuster auf. Erfindungsgemäß konnte überraschenderweise gezeigt werden, daß durch Zellsortierung von Human-Coloncarcinom-Zellinien erhaltene Zellinien ein identisches Zelloberflächenproteinexpressionsmuster von MHC und Zelladhäsionsmolekülen zeigen. Hierbei wurden die typischen Zelladhäsionsmoleküle ICAM (intercellular cellular adhesion molecule), NCAM (neural cellular adhesion molecule) und VCAM (vascular cellular adhesion molecule) sowie MHC Klasse I und Klasse II auf ihre Expression untersucht.

Die erfindungsgemäßen Coloncarcinom-Zellinien unterscheiden sich somit nur in ihrem HSP72-Zelloberflächenexpressionsmuster, während sie in ihrem untersuchten MHC- und Zelladhäsionsmolekül-Zelloberflächenexpressionsmuster identisch sind.

Da die vorliegend beispielhaft bereitgestellten Zellinien CX+ und CX- aus einer homogenen, definierten Mutterzellinie (CX2) abgeleitet wurden, ist der HLA-Hintergrund (alle HLA Allele) identisch; es handelt sich also um ein autologes HLA-Tumorzellsystem.

Erfindungsgemäß wurden somit Zellinien bereitgestellt, die sich hervorragend zur Untersuchung der Funktion von Hitzeschockproteinen der HSP70-Familie eignen.

Die durch Zellseparation gewonnenen Human-Coloncarcinom-Subzellinien, beispielsweise CX+ und CX-, bieten beispielsweise die Möglichkeit, die Bedeutung von HSP (HLA unabhängig) für die Immunogenität von Tumorzellen zu untersuchen. Weiterhin können die erfindungsgemäß bereitgestellten Zellinien, beispielsweise CX+ und CX-, als Feederzellen für NK-Klonierungen und zur Selektion der geeigneten Zellen verwendet werden (NK-Klone mit HSP72-Spezifität). Mit diesen Klonen könnten Rezeptoranalysen durchgeführt werden, um herauszufinden, ob auf NK-Zellen ein HSP72-spezifischer Rezeptor existiert.

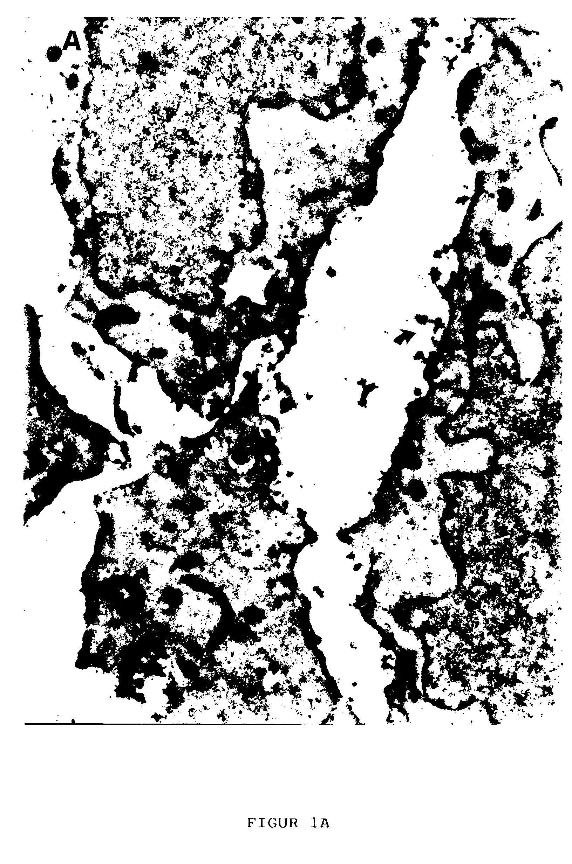

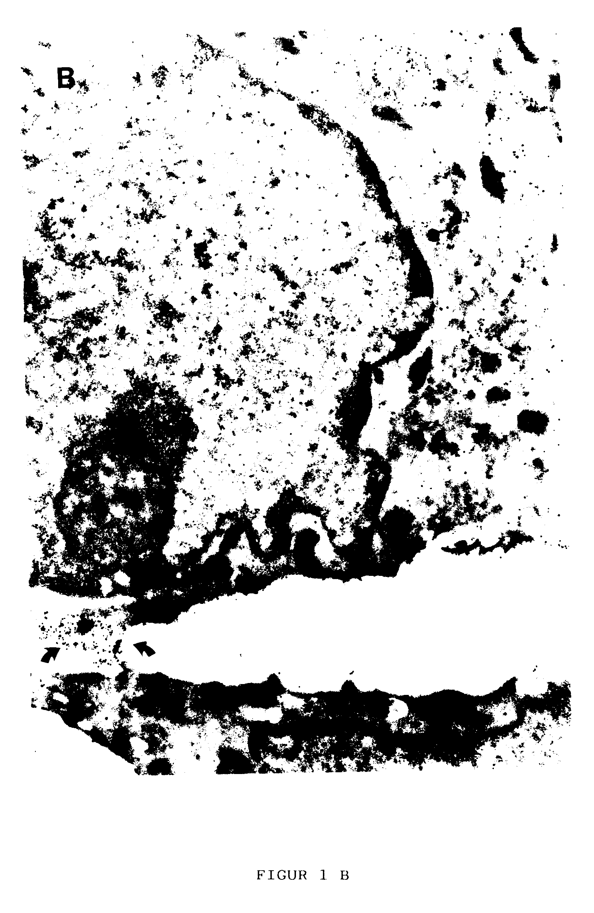

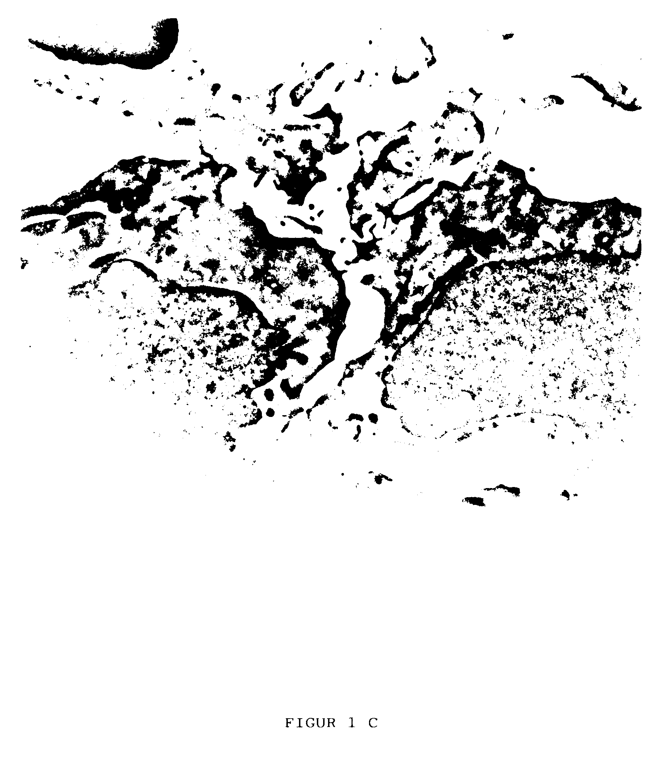

Erfindungsgemäß konnte somit gezeigt werden, daß HSP72 auf der Zelloberfläche von Coloncarcinomzellen exprimiert wird und diese Zellinien durch Zellsortierung in Sublinien aufspaltbar sind, die HSP72 in einer Menge > 80% und < 20% stabil über mehr als 20 Zellpassagen exprimieren. Die Zelloberflächenexpression und die subzelluläre Verteilung von HSP72 wurde durch elektronenmikroskopische Untersuchungen unter Verwendung von ultrakleinen Goldteilchen und Silberfärbung durchgeführt. Die immunelektronenmikroskopische Aufnahme der Figur 1A zeigt, daß HSP72 (schwarze Punkte) mit der Plasamembran von CX2-Coloncarcinomzellen assoziiert ist. Da die Membran vor Antikörperinkubation permeabel war, ist HSP72 auch im Zytoplasma und im Zellkern nachweisbar. Die Ergebnisse der HSP72-Verteilung auf einem unterschiedlichen Abschnitt von zwei Coloncarcinomzellen ist in der Figur 1B dargestellt:
Die Zelle im oberen Teil der elektronenmikroskopischen Aufnahme zeigt eine Anreicherung von HSP72-Molekülen auf Fortsätzen der Plasmamembran und in vesikulären Strukturen. Die Figur 1C zeigt eine Hintergrundsfärbung von drei Coloncarcinomzellen ohne Inkubation mit dem ersten Antikörper.

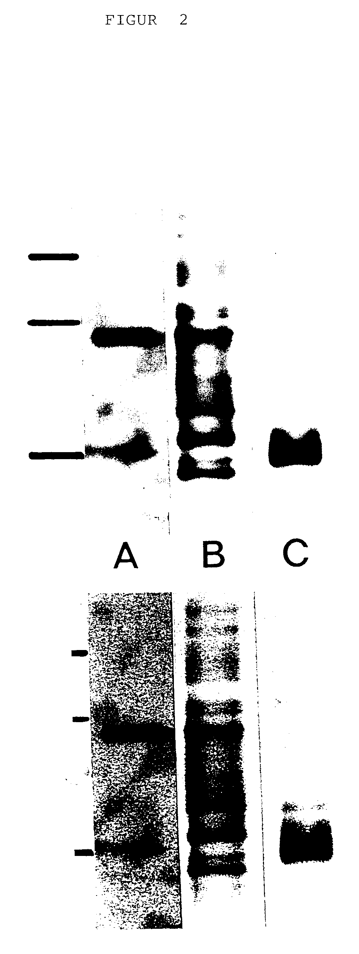

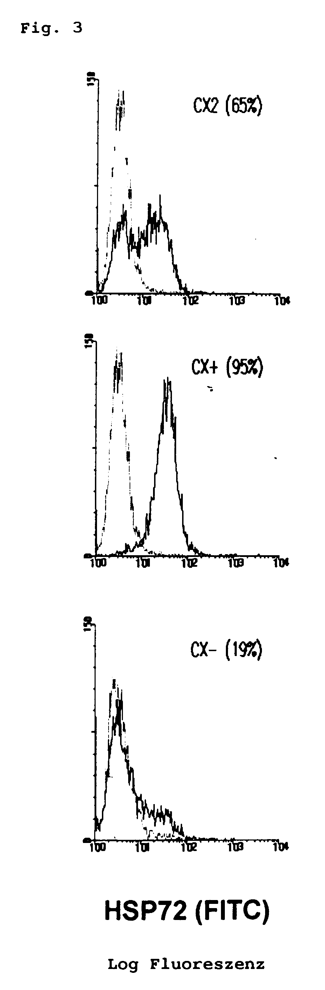

Weiterhin wurde die Zelloberflächenexpression von HSP72 auf CX2-Tumorzellen durch selektive Biotinylierung der Zelloberfläche gezeigt. Ein Vergleich der HSP72 exprimierenden biotinylierten Zelloberfläche mit rekombinantem HSP70 (Fig. 2, Spur A) zeigt, daß ein intaktes HSP72-Molekül von der Zelloberfläche von CX2-Zellen immunpräzipitierbar ist (Fig. 2, Spur B). Die Figur 2, Spur C zeigt eine 45 kDA große Bande, die mit mAB W6/32 immunpräzipitierte Klasse I-MHC-Moleküle darstellt. Eine Quantifizierung der Zelloberflächenexpression von HSP72 auf CX2-Zellen wurde durch indirekte Immunfluoreszenzuntersuchungen unter Verwendung von mAB RPN1197 und 3A3, die für HSP72 spezifisch sind, und nachfolgender FACScan-Analyse durchgeführt. Unter physiologischen Bedingungen wurden die HSP72-Moleküle konstant auf etwa 60% der Zellen exprimiert (vgl. Fig. 3).

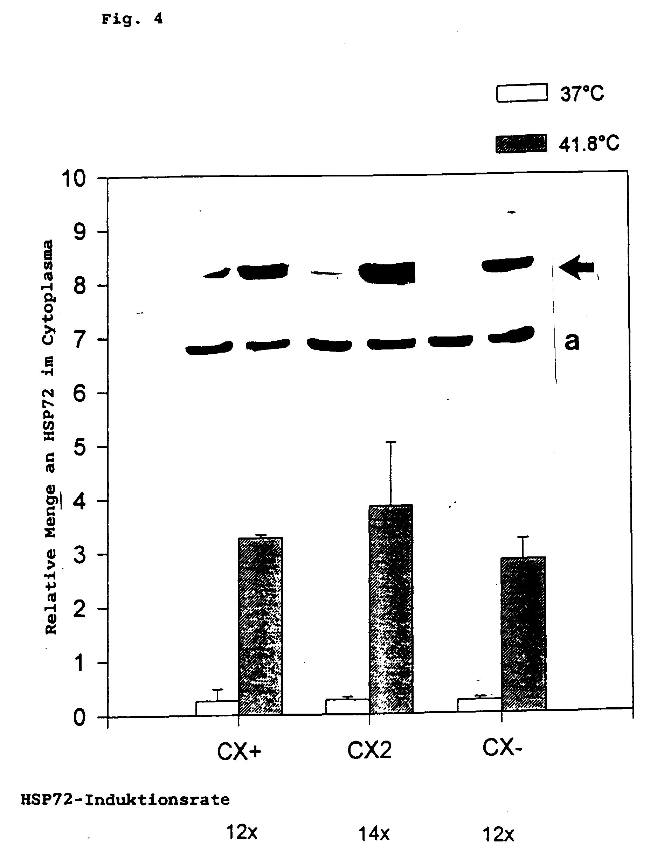

Erfindungsgemäß wurde die CX2-Coloncarcinomzellinie durch Zellsortierung in der in Material und Methoden beschriebenen Weise in eine mehr als 80%, bevorzugt mehr als 90%, HSP72 exprimierende Sublinie und eine weniger als 20%, bevorzugt weniger als 10%, HSP72 exprimierende Sublinie aufgetrennt. Repräsentative Histogramme der phänotypischen Charakterisierung der HSP72-Zelloberflächenexpression auf CX2, CX+ und CX- Zellinien sind in der Figur 4 dargestellt. Durch vergleichende flußzytometrische Analyse von MHC-, Adhäsionsmolekülen und HSP-Expression konnte gezeigt werden, daß sich die CX2-Ausgangszellinie, die CX+ und CX- Sublinien nur in ihrer HSP72-Expressionsfähigkeit auf der Zelloberfläche unterscheiden, nicht dagegen in den anderen untersuchten Zelloberflächenmarkern (vgl. Tabelle 1). Bei vergleichbaren Zelldichten zeigten die CX2, CX+ und CX- Zellen eine vergleichbare ICAM-1-Expression. Dieses Zelloberflächenexpressionsmuster erwies sich über mehr als 30 Zellpassagen stabil.

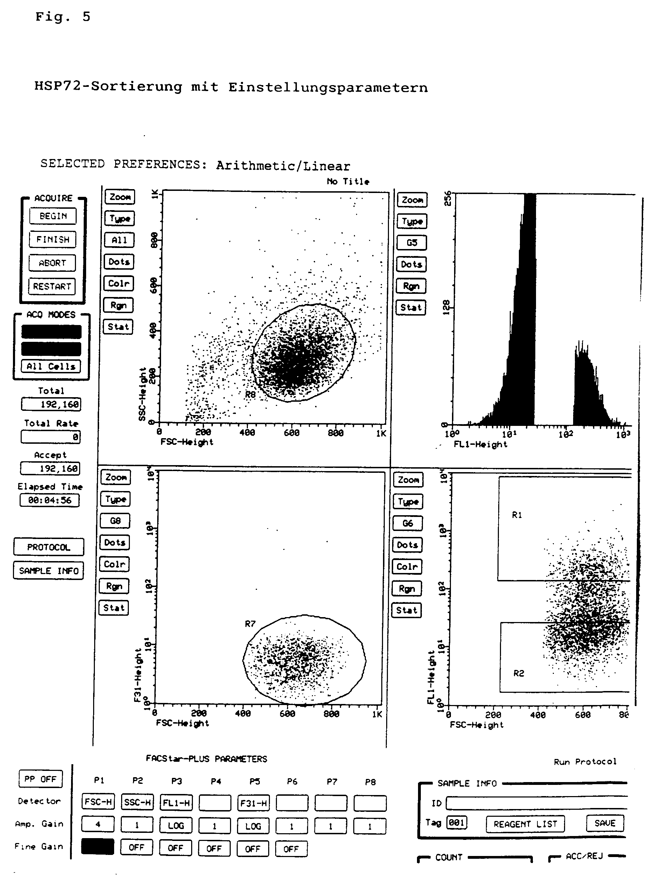

Im Gegensatz zu den deutlichen Unterschieden in der Menge der Expression von HSP72 auf der Zelloberfläche konnte durch Flußzytometrie und Elektronenmikroskopie gezeigt werden, daß der zytoplasmatische Anteil von HSP72 in der CX2-Ausgangszellinie und den Sublinien CX+ und CX- unter physiologischen Bedingungen und nach einer milden Hitzebehandlung (41,8°C, 2 Stunden) vergleichbar war. Wie die Figur 5 zeigt, ergibt ein milder Hitzeschock in allen drei Zellinien eine vergleichbare Induktionsrate von HSP72 (etwa 13-fach).

Erfindungsgemäß konnte somit überraschend gezeigt werden, daß Human-Coloncarcinom-Zellinien eine Zelloberflächenexpression von HSP72 von etwa 60% der Zellen bereits unter physiologischen Bedingungen aufweisen. Diese unerwartete HSP72-Zelloberflächenexpression auf Coloncarcinom-Zellen bildete die Grundlage für die erfindungsgemäß durchgeführte Aufsplittung in zwei CX2-Coloncarcinom-Sublinien, die HSP72 zu mindestens mehr als 80% (CX+) und zu weniger als 20% (CX-) der Zellen exprimieren. Die Auftrennung in Sublinien wurde aufgrund der Expression von HSP72 auf der Zelloberfläche der Coloncarcinom-Zellinien durch Zellsortierung unter Verwendung geeigneter Antikörper durchgeführt. Überraschenderweise zeigte sich bei der durchgeführten Flußzytometrieanalyse, daß das Zelloberflächenexpressionsmuster von HSP72 in der CX2-Ausgangszellinie und den zwei Sublinien CX+ und CX- über mehr als 30 Zellpassagen stabil blieb. Auch ein Hitzestreß beeinflußte das Oberflächenexpressionsmuster nicht. Trotz dieser deutlichen Unterschiede in der HSP72-Expression wiesen die Zellen bei vergleichbaren Zelldichten ein vergleichbares und konstantes Expressionsmuster von MHC- und Adhäsionsmolekülen auf.

Das erfindungsgemäß bereitgestellte "autologe" Tumorzellsystem unterscheidet sich somit-nur in der Expression eines Antigens (HSP72), während die Expression von MHC und Zelladhäsionsmolekülen identisch ist.

In der Expressionsrate und in der Menge des exprimierten HSP72 konnten auf der zytoplasmatischen Ebene keine Unterschiede festgestellt werden. Nach Hitzeexposition konnte die Expression von HSP72 im Zytoplasma in beiden Zellinien gleichermaßen induziert werden. Auch bezüglich der Wachstumsparameter und der Morphologie der Zellinien bestehen keine signifikanten Unterschiede. Die Verdopplungszeit bei beiden Zellinien beträgt ca. 25 Stunden. Die Überlebensfähigkeit nach Hitzeexposition oder nach Einwirkung anderer Streßfaktoren ist für beide Zellinien vergleichbar. Ebenfalls wurde eine vergleichbare Thermotoleranz gegenüber Temperaturen im Bereich von 40°C - 44°C festgestellt.

Erfindungsgemäß wird somit zum ersten Mal ein System zur Verfügung gestellt, um die Rolle von HSP72 als tumorspezifische Erkennungsstruktur in einem autologen Tumorzellsystem zu untersuchen.

Es konnte weiterhin gezeigt werden, daß die hitzeunabhängige Expression von HSP72 auf der Zelloberfläche von CX2 und CX+-Coloncarcinomzellen eng mit der Sensitivität dieser Zellen zur Lyse durch NK-Zellen korreliert. Dies zeigt, daß neben HSP72 kein weiterer hitzeinduzierbarer Faktor zur Erkennung durch die NK-Zellen notwendig ist.

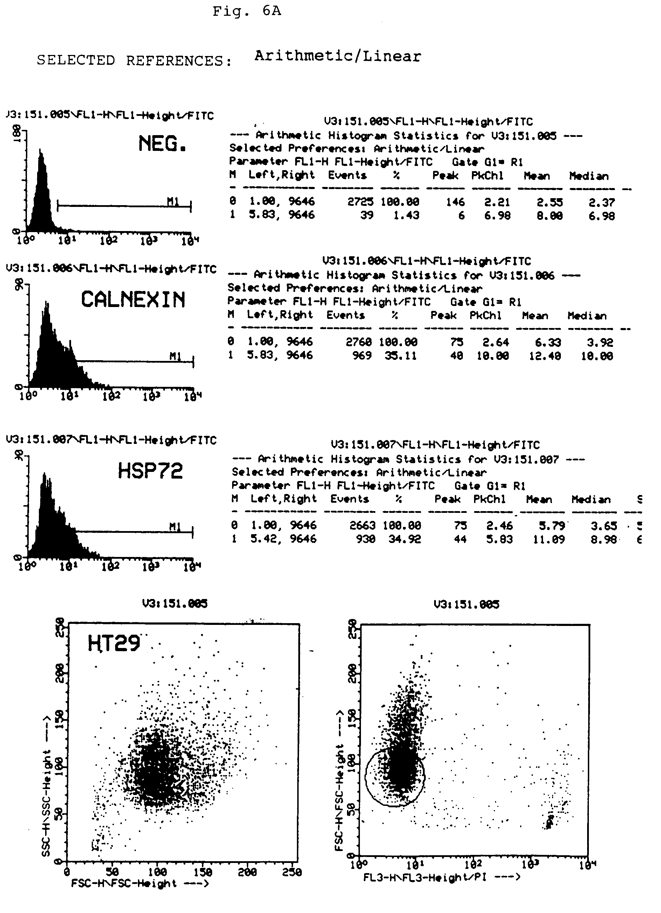

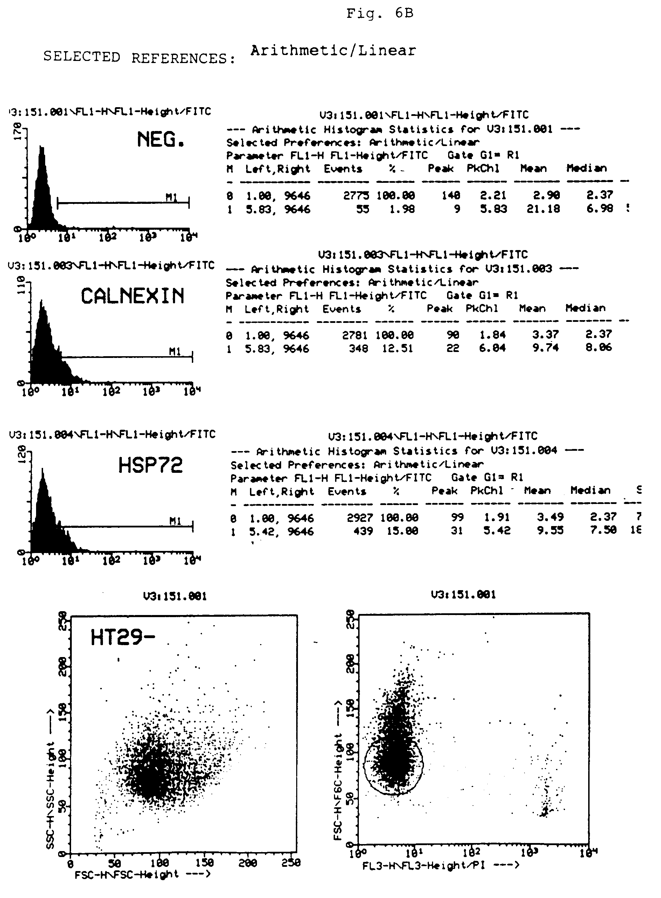

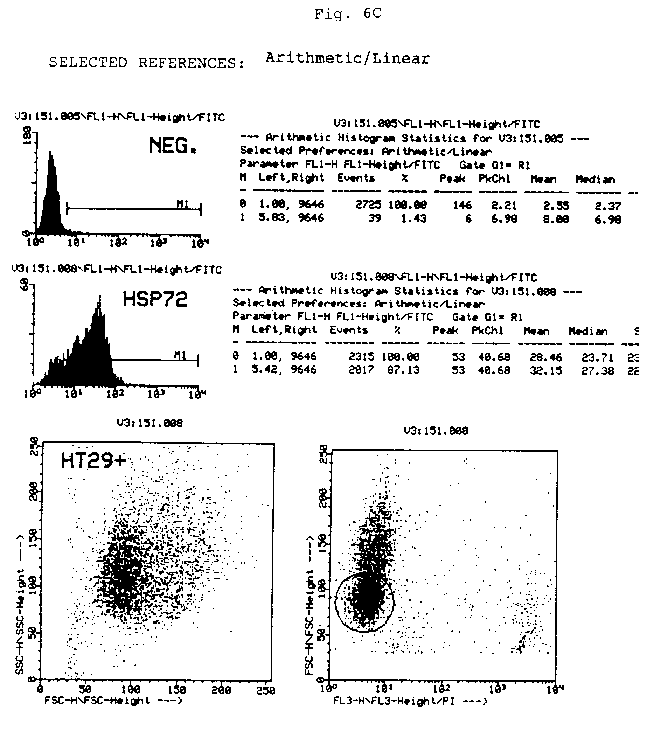

Die gesamte Länge von HSP72 beträgt etwa 700 Aminosäuren. Am N-Terminus liegen die Aminosäuren 1 - 450, am C-Terminus die Aminosäuren 450 - 650 (vgl. Fig. 6). Die Aminosäure-Sequenz von HSP72 ist an sich bekannt und ist beispielsweise in Hunt + Morimoto, Proc.Natl.Acad.Sci.USA, 82 (1985), 6455-6459, veröffentlicht.

Erfindungsgemäß wurde weiterhin die Rolle der differentiellen Expression des Zelloberflächenproteins HSP72 in diesem "autologen" Colon-Carcinom-Zellsystem in Bezug auf seine Sensitivität gegen durch NK-Zellen vermittelte Lyse untersucht. Es konnte gezeigt werden, daß die Lyse durch NK-Zellen in der HSP72 in hoher Menge exprimierenden CX+ Sublinie um mehr als das Zweifache erhöht war im Vergleich zu der HSP72 in geringer Menge exprimierenden CX- Sublinie. Die Lyse-Sensitivität der Ausgangs-Tumor-Zellinie CX2 lag in etwa zwischen den CX+ und CX- Zellen. Weiterhin konnte gezeigt werden, daß ein Hitzeschock weder eine erhöhte Zelloberflächenexpression von HSP72 noch eine erhöhte Sensitivität gegen Lyse durch NK-Zellen bewirkt.

Durch Antikörper-Blockierungstests konnte die durch NK-Zellen vermittelte Lyse-Sensitivität mit der HSP72-Menge korreliert werden, die an der Zelloberfläche exprimiert wird. Unter Verwendung des für HSP72 spezifischen monoklonalen Antikörpers wurde die stärkste Inhibierung bei CX+ Zellen gefunden, eine mittlere Lyse-Inhibierung bei der ursprünglichen CX2-Tumorzellinie und keine Lyse-Inhibierung bei der CX- Zellsublinie. Ein Kontrollantikörper vom IgG1-Isotyp und ein MHC-Klasse-I-spezifischer Antikörper zeigte keinen Inhibierungseffekt auf das Lysemuster bei den drei genannten Tumorzellinien CX2, CX+ und CX-.

Wie bereits ausgeführt, unterscheidet sich das "autologe" Tumorzellsystem nur in der Expression eines einzigen Antigens; damit wird erfindungsgemäß die Untersuchung der Rolle von HSP72 als tumorspezifische Erkennungsstruktur ermöglicht. Es konnte in der Literatur (16) gezeigt werden, daß Mitglieder der HSP70-Familie sowohl in vivo als auch in vitro eine zelluläre Immunantwort hervorrufen. Deshalb wurde erfindungsgemäß die Lyse-Sensitivität der erfindungsgemäßen "autologen" Coloncarcinom-Sublinien, die sich nur in ihrer HSP72-Expression auf der Zelloberfläche unterscheiden, miteinander verglichen. Für die Zytotoxizitäts-Untersuchungen bei diesen Coloncarcinom-Sublinien wurde eine mit NK angereicherte Effektor-Zellpopulation verwendet. Die hitzeunabhängige Zelloberflächenexpression von HSP72 auf CX2 und CX+ Coloncarcinomzellen korrelierte eng mit der durch NK-Zellen vermittelten Lyse-Sensitivität. Diese Daten zeigen, daß neben HSP72 kein zusätzlicher hitzeinduzierbarer Faktor für den durch NK-Zellen vermittelten Erkennungsmechanismus notwendig ist.

Die Expression von HSP72 ist damit ein zusätzlicher Faktor, der die Sensitivität von Tumor-Targetzellen für eine durch NK-Zellen vermittelte Lyse bestimmt. Die Expression von HSP72 könnte somit bei der Tumortherapie verwendet werden, um NK-Zellen zu stimulieren.

**TABELLE 1**

| Vergleichende durchflußzytometrische Analyse von MHC, Adhäsionsmolekülen und HSP72-Zelloberflächenexpression auf CX2, CX+ und CX- Carcinomzellen (n = 5). | | | | | | | |
|---|---|---|---|---|---|---|---|
| | MHC I | MHC II | ICAM-1 | VCAM | HNK1 | NCAM | HSP72 |
| | W6/32 | L243 | anti-CD54 | anti-CD106 | anti-CD57 | anti-CD56 | anti-HSP72 |
| CX2 | +++ (99±0.5) | -(5±3.7) | ++ (58±2.3) | -(8±0.5) | -(7±1.5) | + (21±4.6) | ++ (66±6.8) |
| CX+ | +++ (99±0.8) | -(10±0.5) | ++ (68±7.7) | -(11±3.5) | -(12±6.6) | + (26±2.3) | +++ (93±3.4) |
| CX- | +++ (99±0.5) | -(10±4.7) | ++ (48±4.5) | -(12±4.5) | -(10±4.0) | + (24±8.8) | -(18±6.0) |
| +++: > 90% ++: > 50% + : > 20% - : < 20% | | | | | | | |

### MATERIAL und METHODEN

### ZELLKULTUR

Die Human-Coloncarcinom-Zellinie CX2 (Tumorbank DKFZ Heidelberg) und die Subklone CX+ und CX- wurden in RPMI 1640 Medium (Gibco, Eggenstein), supplementiert mit 10% hitze-inaktiviertem fötalen Kälberserum (FCS, Gibco, Eggenstein) und Antibiotika, kultiviert.

### VORBEREITUNG DER CX2-TUMORZELLEN ZUR SEPARATION MITTELS FACS:

Zunächst wurden die zu analysierenden Zellen zweimal mit kaltem PBS/10% FKS-Medium gewaschen. Das Zellpellet (5 x 10⁶ Zellen) wurde resuspendiert und 30 Minuten auf Eis mit 5 µl RPN1197 (anti-HSP72 mAk; Amersham; CODE RPN 1197) inkubiert. Um die nach dieser Inkubationsphase nicht an die Zellen gebundenen Antikörper zu entfernen, wurden die Zellen erneut zweimal mit kaltem PBS/10% FKS-Medium gewaschen. Daraufhin wurden diese Zellen mit 5 µl eines Fluoreszeinisothiocyanat- (FITC)-konjugierten Kaninchen-anti-Maus-Antikörpers (DAKO; CODE No. F0232) 30 Minuten bei Dunkelheit auf Eis inkubiert. Überschüssiger FITC-Antikörper wurde durch zweimaliges Waschen entfernt, anschließend die Zellen in 2 ml kaltem PBS/10% FKS-Medium resuspendiert und bis zur Separation im Dunkeln auf Eis gelagert. Zur Unterscheidung lebender und toter Zellen wurde den Proben 10 µl Propidiumiodid (PI; 50 µg/ml) zugegeben. Mit Hilfe des FACS-Star-Sorters wurde die "Mutterzell-Linie" CX2 aufgrund der Fluoreszenzmarkierung in 2 Tochterlinien aufgetrennt: eine CX-Linie, die weniger als 20% HSP72 auf der Zelloberfläche exprimiert bzw. eine CX+Linie, die mehr als 80% HSP72 exprimiert.

### HITZEBEHANDLUNG

Exponentiell wachsende CX2-Zellen werden bei der nicht-letalen Temperatur von 41,8°C zwei Stunden lang in einem temperaturkontrollierten Wasserbad (Haake E3, Karlsruhe) behandelt und dann bei 37°C zwölf Stunden lang inkubiert. Die Überlebensrate der hitzebehandelten Zellen betrug immer über 95%, bestimmt durch Trypan-Blau-Ausschluß und PI-Färbung.

### MONOKLONALE ANTIKÖRPER (mAb), INDIREKTE IMMUNFLUORESZENZ- und FACScan-ANALYSE:

Die nachfolgenden Antikörper wurden zur phänotypischen Charakterisierung von Effektor- und Tumorzellen auf einem FACScan-Instrument (Becton Dickinson, Heidelberg) zur Zellsortierung* auf einen FACS-Stab-Plus-Instrument (Becton Dickinson, Heidelberg), für Antikörper-Blockierungs-Untersuchungen** und zur Immunpräzipitation*** verwendet (Methoden wie unten beschrieben)

| **Antikörper** | **Spezifität** | **Isotyp** | **Herkunft** |
|---|---|---|---|
| Kontrolle | isotype ctrl. | IgG2a | Dianova**, Hamburg, FRG |
| Kontrolle | isotype ctrl. | IgG1 | Dianova |
| W6/32 | MHC class I | IgG2a | J. Johnson**^{,}***, LMU Munich, FRG |
| L243 | HLA DR | IgG2a | J. Johnson |
| anti-CD54 | I-CAM | IgG1 | Dianova |
| anti-CD56 | N-CAM | IgG1 | Dianova |
| anti-CD16 | Fcy RIII | IgG1 | Dianova |
| anti-CD57 | HNK1 | IgM | Dianova |
| VCAM-1 | VCAM | IgG1 | Dianova |
| anti-CD3 | T | IgG1 | Dianova |
| OKT3 | T | IgG2a | ATCC* |
| anti-CD14 | Monocyten | IgG2a | Dianova* |
| RPN1197 | HSP72 | IgG1 | Amersham, Braunschweig, FRG*^{,}**^{,}*** |
| 3A3 | HSP72 | IgG1 | S. Fox, NW University, Illinois, USA |
| CD3/CD16+56 | T/NK | IgG1/IgG1 | Becton-Dickinson, Heidelberg, FRG |
| CD45/CD14 | lymph. | IgG1/IgG2b | Becton Dickinson |
| FITC rabbit anti-mouse | | Ig | Dako |

Die durchflußzytometrischen Untersuchungen wurden mit exponentiell wachsenden Zellen und bei vergleichbaren Zelldichten durchgeführt. Lebensfähige Zellen (1x10⁶) wurden mit einem der oben gezeigten Antikörper in eine Endkonzentration von 1-5 µg/0,5-1x10⁶ Zellen 30 Minuten lang bei 4°C inkubiert. Die zur FACScan-Analyse und zum Zellsortieren verwendeten Antikörper enthielten 0,1% Natriumazid; die für die Antikörper-Blockierungs-Untersuchungen verwendeten Antikörper enthielten kein Natriumazid. Nach Inkubation mit den primären, ungefärbten Antikörpern wurden die Zellen zweimal mit PBS/10% FKS-Lösung gewaschen und mit einem zweiten, Fluoreszeinisothiocyanat-konjugierten (FITC) Kaninchen-anti-Maus Ig-Antikörper (FITC, DAKO, Hamburg) für weitere 30 Minuten bei 4°C inkubiert. Der Prozentsatz an positiv gefärbten Zellen wurde als Differenz der Anzahl spezifisch gefärbter Zellen minus der Anzahl der Zellen, die mit an den Isotyp angepaßten Kontrollantikörpern gefärbt wurden, definiert. Die aus der FACScan-Analyse erhaltenen Werte repräsentieren Mittelwerte aus zumindest 4 unabhängigen Versuchen.

Die Spezifität des HSP72 mAB RPN1197 (Amersham) Antikörpers, der bei den vorliegenden Untersuchungen verwendet wurde, wurde bereits früher (5) durch Western-Blotting nach 2D und 1D SDS-PAGE gezeigt: Der Antikörper weist selektiv HSP72 nach und kreuzreagiert nicht mit HSP73. HSP70-Zytoplasmaexpression war mit den nachfolgenden Antikörpern nachweisbar: RPN1197 (Amersham), 3A3 (S. Fox), C92F3A-5 (StressGen) und 5G10 (Pharmagen). Eine Zelloberflächenfärbung und Zellsortierung der Tumorzellen wurde mit mAb RPN1197 (Amersham) durchgeführt. Die HSP72 hoch (CX+) und gering (CX-) exprimierenden Coloncarcinom-Sublinien wurden nach der Zellsortierung getrennt kultiviert.

Die Zellsortierung der Effektorzellen wurde über CD3 und CD14 durchgeführt. Die negativ aufgetrennte CD3-Effektorzell-Population wurde mit 100 IU rIL-2 stimuliert und für Funktionsuntersuchungen verwendet. Die phänotypische Charakterisierung dieser Zellen wurde unter Verwendung von CD3/CD16+56 und CD14/CD45 doppelt gefärbten Antikörpern durchgeführt.

### ELEKTRONENMIKROSKOPIE

Mit PBS gewaschene CX2-Tumorzellen wurden in 8% Paraformaldehyd in Hepes(250 mM)-Puffer 1 Stunde lang fixiert. Nach zwei Waschungen wurden freie Aldehydgruppen in 50mM NH₄Cl für 10 Minuten gelöscht. Die Zellpellets wurden in einem Volumen von 2,1 M Saccharose in Polyvinylpyrrolidon (17%) bei 20°C 30 Minuten lang zum Kryoschutz in suspendierter Form aufbewahrt. Dann wurden die Zellpellets in flüssigem Stickstoff eingefroren, und ultradünne Schnitte (70 nm) wurden bei -100°C auf einem Ultracut-Mikrotom (Reichert-Jung FC4E) unter Verwendung eines Glasmessers abgeschnitten und auf 150-mesh-Gittern aus Parlodionbeschichtetem Nickel befestigt. Die Immun-Gold-Markierung von HSP72 wurde unter Verwendung von ultrakleinen Goldproben und nachfolgender Silberverstärkung nach dem von Danscher beschriebenen Verfahren (12) durchgeführt.

### SELEKTIVE BIOTINYLIERUNG DER ZELLOBERFLÄCHE und IMMUNPRÄZIPITATION

Zum Nachweis der Zelloberflächenexpression von HSP72 wurden lebensfähige Zellen (2x10⁷) mit Biotinester (RPN2202, Amersham) 30 Minuten lang bei 4°C biotinyliert. Nach dem Waschen wurden die Zellen 20 Minuten lang in eiskaltem Lysepuffer (0,1% NP40; 250 mM NaCl; 25 mM Tris-HCl, pH 7,5; 5 mM EDTA, 2 µg/ml Aprotinin, 100 µg/ml PMSF) lysiert. Die Extrakte wurden durch Mikrozentrifugation 15 Minuten lang bei 4°C gereinigt und mit HSP72 (10 µl) oder mit W6/32 nAb (2 µg) und 50 µl gamma-Bind plus Protein G-Sepharose-Kügelchen 3 Stunden lang bei 4°C immunpräzipitiert. Dann wurden die konjugierten, mit dem Antigen beladenen Sepharosekügelchen dreimal im eiskalten Lysepuffer gewaschen und in 75 µl Probenpuffer (200 mM DTT; 20% Glycerin; 0,12 M Tris; Bromphenol-Blau) resuspendiert. Die Proteine aus den Sepharosekügelchen wurden auf 10% SDS-PAGE aufgetrennt, auf eine PVDF-Membran geblottet und durch ECL nach Inkubation mit einem sekundären Peroxidase-konjugierten Streptavidin-Antikörper nachgewiesen.

### SDS-PAGE und WESTERN BLOT ANALYSE

Gleiche Proteinmengen (10 µg) von Zellysaten und 50 ng rHSP70 (StressGen, Victoria, Canada) wurden auf 10% SDS-PAGE, wie in (5) beschrieben, gemäß dem Verfahren von Laemmli (13) einer Elektrophorese unterzogen. Nach der SDS-PAGE wurden die Proteine auf Immobilon-PVDF-Membrane (Millipore Corp., Bedford, MA) gemäß einem Standardprotokoll (14) übertragen. Die unspezifische Bindung der Immobilon-PVDF-Membrane wurde mit 5% Magermilch in PBS blockiert. Die Blots wurden mit dem primären HSP72-Antikörper (Amersham) und dem sekundären Antikörper (Ziege-anti-Maus IgG Peroxidase konjugiert, BioRad) je 1 Stunde lang inkubiert. Unter Verwendung des ECL Western-Blotting-Nachweissystems (Amersham, Braunschweig) wurden Immunkomplexe nachgewiesen. Für biotinylierte Proben wurden 20 µl der Proben einer Elektrophorese unterzogen und auf Immobilon PVDF-Membrane (Millipore Corp., Bedford, MA) übertragen. Die Membranen wurden 1 Stunde lang bei Raumtemperatur in 5% Blockierungsmittel (Amersham, NIF833) und 1% Tween-20 (Sigma) enthaltendem PBS blockiert, dreimal für 10 Minuten je in 1% Tween-20 (PBS-T) enthaltendem PBS gewaschen und 1 Stunde lang mit Meerrettichperoxidase-konjugiertem Streptavidin (Amersham), verdünnt 1:1500 in PBS-T, markiert. Die biotinylierten Proteine wurden durch das ECL-System nachgewiesen.

### ANTIKÖRPER-BLOCKIERUNGS-UNTERSUCHUNGEN

Die Inhibierungstests wurden durch Vorinkubation der Targetzellen mit den nachfolgenden Antikörpern in einer Endkonzentration von 5 µg/1x10⁶ Zellen für jeden Antikörper durchgeführt: RPN1197 (anti-HSP72), ein an den Isotyp angepaßter IgG1-Kontroll-Antikörper und W6/32 (15). Nach Inkubation wurden die Zellen zweimal mit RPMI1640/10% FKS gewaschen, und der Zytotoxizitätstest wurde, wie oben beschrieben, durchgeführt.

Die für die Zellinie CX2 erhaltenen Ergebnisse ließen sich auch für die Human-Coloncarcinom-Zellinien HT29 bestätigen. Die Zellinie HT29 ist kommerziell bei der AMERICAN TISSUE TYPE CULTURE COLLECTION unter der Nummer ATCC HTB38 erhältlich. Die genotypische Beschreibung ist aus dem ATCC-Katalog und der dort angeführten Literatur ersichtlich.

Die beiliegenden Figuren 9A bis C zeigen die Ergebnisse der Zellsortierung von HT29. Der Versuch wurde entsprechend der Zellsortierung der CX2-Zellinie durchgeführt.

Die HT29-Ursprungszellinie exprimiert auf ihrer Zelloberfläche HSP72 in einer Menge von 30 bis 40% der Zellen. Durch Zellsortierung konnte eine HT29+ Zellinie erhalten werden, die auf ihrer Zelloberfläche HSP72 in 87% der Zellen exprimiert, und eine HT29- Zellinie, die HSP72 in 15% der Zellen exprimiert. Die erhaltenen HT29-Sublinien zeigen eine stabile HSP72-Expression über zumindest 20 Zellpassagen sowie ein identisches MHC- und Zelladhäsionsmolekül-Zelloberflächenexpressionsmuster.

Aufgrund dieser Untersuchungen konnte gezeigt werden, daß es sich nicht um einen Spezialfall bei CX2-Zellinien handelt, sondern daß eine Verallgemeinerung auf Human-Coloncarcinom-Zellinien in ihrer Gesamtheit möglich ist.

### Literatur

1. Welch, W. J. 1992. Mammalian stress response: cell physiology, structure/function of stress proteins, and implications for medicine and disease. *Physiol*. *Rev*. 72:1063-1081.
2. Craig, E. A., B. D. Gambill, and R. J. Nelson. 1993. Heat shock proteins: molecular chaperones of protein biogenesis. *Microbiol*. *Rev*. 57:402-414.
3. Welch, W. J., and J. R. Feramisco. 1984. Nuclear and nucleolar localization of the 72,000-dalton heat shock protein in heat-shocked mammalian cells. *J Biol*. *Chem*. 259:4501-4513.
4. Ferrarini, M., S. Heltai, M. R. Zocchi, and C. Rugarli. 1992. Unusual expression and localization of heat-shock proteins in human tumor cells. *Int*. *J Cancer* 51:613-619.
5. Multhoff, G., C. Botzler, M. Wiesnet, E. Müller, T. Meier, W. Wilmanns, and R. D. Issels. 1995. A stress-inducible 72-kDa heat-shock protein (HSP72) is expressed on the surface of human tumor cells, but not on normal cells. *Int*. *J Cancer* 61:272-279.
6. Lamb, J. R., V. Bal, J. B. Rothbard, A. Mehlert, P. Mendez-Samperio, and D. B. Young. 1989. The mycobacterial GroEL stress protein: a common target of T-cell recognition in infection and autoimmunity. *J Autoimmun*. 2 Suppl:93-100.
7. Heufelder, A. E., B. E. Wenzel, and R. S. Bahn. 1992. Cell surface localization of a 72 kilodalton heat shock protein in retroocular fibroblasts from patients with Graves' ophthalmopathy. *J Clin*. *Endocrinol*. *Metab*. 74:732-736.
8. Jacquier-Sarlin, M. R., K. Fuller, A. T. Dinh-Xuan, M. J. Richard, and B. S. Polla. 1994. Protective effects of hsp70 in inflammation. *Experientia* 50:1031-1038.
9. Polla, B. S., S. Kantengwa, G. J. Gleich, M. Kondo, C. M. Reimert, and A. F. Junod. 1993. Spontaneous heat shock protein synthesis by alveolar macrophages in interstitial lung disease associated with phagocytosis of eosinophils. *Eur*. *Respir*. *J* 6:483-488.
10. Ullrich, S. J., E. A. Robinson, L. W. Law, M. Willingham, and E. Appella. 1986. A mouse tumor-specific transplantation antigen is a heat shock-related protein. *Proc*. *Natl*. *Acad*. *Sci*. *U*. *S*. *A*. 83:3121-3125.
11. Srivastava, P.K. 1994. Heat shock proteins in immune response to cancer: the Fourth Paradigm. Experientia 50:1054-1060.
12. Danscher, G. 1981. Localization of gold in biological tissue. A photochemical method for light and electronmicroscopy. Histochemistry 71:81-88.
13. Laemmli, U.K. 1970. Cleavage of structural proteins during the assembly of the head of bacteriophage T4. Nature 227:680-685.
14. Towbin, H., T. Staehelin, and J. Gordon. 1979. Electophoretic transfer of proteins from polyacrylamide gels to nitrocellulose sheets: procedure and some applications. Proc. Natl. Acad. Sci. U.S.A. 76:4350-4354.
15. Parham, P., M.J. Androlewicz, F.M. Brodsky, N.J. Holmes, and J.P. Ways. 1982. Monoclonal antibodies: purification, fragmentation and application to structural and functional studies of class I MHC antigens. J Immunol. Methods 53:133-173.
16. Gomez, F. J., A. M. Gomez, and G. S. J. Deepe. 1992. An 80-kilodalton antigen from Histoplasma capsulatum that has homology to heat shock protein 70 induces cell-mediated immune responses and protection in mice. *Infect*. *Immun*. 60:2565-2571.

## Patentansprüche

1. Human-Coloncarcinom-Zellinien, die eine stabile HSP72-Expression von > 80% oder < 20% und ein im wesentlichen identisches Zelloberflächenproteinexpressionsmuster von MHC und Zelladhäsionsmolekülen zeigen.

2. Human-Coloncarcinom-Zellinie nach Anspruch 1, dadurch gekennzeichnet, daß sie von einer CX2- oder HT29-Zellinie abgeleitet ist.

3. Human-Coloncarcinom-Zellinie nach Anspruch 1 oder Anspruch 2, dadurch gekennzeichnet, daß sie eine HSP72-Expression von > 90% oder < 10% aufweist.

4. Human-Coloncarcinom-Zellinie CX+ mit einer stabilen Expression von HSP72 an der Zelloberfläche von > 80%, bevorzugt > 90%, mit der Zugangsnummer DSM ACC 2287.

5. Human-Coloncarcinom-Zellinie CX- mit einer stabilen Expression von HSP72 an der Zelloberfläche von < 20%, bevorzugt < 10%, mit der Zugangsnummer DSM ACC 2288.

6. Human-Coloncarcinom-Zellinie nach einem der Ansprüche 1-5 mit einem einheitlichen Zelloberflächenexpressionsmuster der Zelladhäsionsmoleküle ICAM, NCAM und VCAM.

7. Verfahren zur Herstellung von Human-Coloncarcinom-Zellinien nach einem oder mehreren der vorhergehenden Ansprüche,
dadurch gekennzeichnet, daß
eine Human-Coloncarcinom-Zellinie, die HSP72 auf ihrer Zelloberfläche exprimiert, durch Zellsortierung in zwei Sublinien aufgetrennt wird, wobei die eine Sublinie HSP72 zu mehr als 80% und die andere Sublinie HSP72 zu weniger als 20% exprimiert.

8. Verwendung der Human-Coloncarcinom-Zellinien nach einem oder mehreren der vorhergehenden Ansprüche zur Untersuchung der Funktion von HSP72 bei der Tumorentstehung und Tumortherapie.
